# EUROPÄISCHE PATENTSCHRIFT

(11) **EP 2 977 106 B1**
(45) Veröffentlichungstag und Bekanntmachung des Hinweises auf die Patenterteilung: **18.12.2019**
(21) Anmeldenummer: 15182541.1
(22) Anmeldetag: 26.02.2013
(51) Int. Cl.: B02C 19/06, B02C 23/02, B02C 23/08, B03B 9/06, C12M 1/33, C10L 5/44, D21B 1/06

(54) **VERFAHREN UND VORRICHTUNG ZUR GROSSTECHNISCHEN AUFBEREITUNG VON BIOMASSE**
METHOD AND DEVICE FOR LARGE-SCALE PROCESSING OF BIOMASS
PROCEDE ET DISPOSITIF DESTINES AU TRAITEMENT DE BIOMASSE A L'ECHELON INDUSTRIEL

(30) Priorität: 29.02.2012 DE 102012203148
(43) Veröffentlichungstag der Anmeldung: 27.01.2016
(62) Teilanmeldung aus: 13706021.6
(73) Patentinhaber: Werner, Hans, 81825 München (DE)
(72) Erfinder: WERNER, Hans, 81825 München (DE); SCHLEDERER, Swantje Mignon, 85567 Grafing (DE)
(74) Vertreter: Leske, Thomas

(56) Entgegenhaltungen:
- EP-A2- 0 521 685
- EP-A2- 2 500 112
- DE-U1-202004 008 685
- PL-B1- 199 667
- US-A- 3 680 796

## Beschreibung

Die Anmeldung betrifft ein Verfahren und eine Vorrichtung zur großtechnischen Aufbereitung von Biomasse, wobei die Biomasse mindestens einem Zerkleinerungsschritt unterworfen wird.

Biomasse wie beispielsweise Halmgut (Gras), Laub, Zweige, dünne Äste, Grünschnitt im Allgemeinen, sonstige pflanzliche Abfälle, Landschaftspflegematerial wie Straßenbegleitgrün, landwirtschaftliche Reststoffe im Allgemeinen, beispielsweise Mist und sonstige Biorestabfälle bis hin zu Klärschlamm werden herkömmlich kompostiert.

Mit der Zunahme des Problembewusstseins, was die Nutzung fossiler Brennstoffe und der Atomenergie angeht, rücken sie neben der Verwendung als Energiequellen auch zur Gewinnung von Fasern für die Papierindustrie gemäß US 3 680 796 und PL 199 667 B1 durch Aufbereitung mittels eines Zerkleinerungsschrittes mittels Wasserschneider in das Bewusstsein.

Diese Verwendungszwecke setzen eine Aufbereitung der Biomasse voraus, die einen Zerkleinerungsschritt einschließt. Das Zerkleinern ist nicht nur wesentlich für die spätere Handhabung. Sie ist vielmehr in den verschiedenen erwähnten Verwertungsarten in vielfältiger Hinsicht von Bedeutung, um die Biomasse für die weitere Verarbeitung und Nutzung zu optimieren.

So ist es bei der Aufbereitung von Biomasse in verschiedenen Aufbereitungsverfahren ganzwesentlich, dass der in ihr enthaltene Wassergehalt auf möglichst energiesparende Weise verringert werden kann. Deshalb wird dem Zerkleinern häufig ein mechanisches Pressen nachgeschaltet, das deutlich weniger energieaufwändig ist als das alternativ in Betracht zu ziehende thermische Trocknen und dessen Effizienz deutlich steigerbar ist, wenn die Biomasse zuvor zerkleinert worden, die Zellstruktur also bereits weitgehend eröffnet, d.h. aufgeschlossen ist.

Auch dort, wo zunächst keine möglichst trockene Masse hergestellt werden soll, sondern eine mehr oder weniger feuchte Masse benötigt wird, wird dem durch den mit dem Zerkleinern verbundenen Austritt von Zellflüssigkeit Vorschub geleistet.

Hinzu kommt, dass mit der beim Zerkleinern austretenden Zellflüssigkeit die darin enthaltenen verbrennungsschädlichen Stoffe wie Cl, K, Ca, Mg etc., die u.a. in der Zellflüssigkeit gelöst sind, aus der Festmasse besser entfernt werden.

Dies geschieht in verstärktem Maße, wenn der Biomasse zusätzlich eine Flüssigkeit, etwa Wasser zum Waschen oder eine Prozessflüssigkeit wie etwa eine gärfördernde Lösung oder eine Mischung daraus hinzugefügt wird. Dann nämlich werden die wasserlöslichen Stoffe/Elemente regelrecht aus den Zellen ausgewaschen. Die darin gelösten verbrennungsschädlichen Substanzen gelangen in die Flüssigkeit des Gemischs und werden spätestens dann großteils aus der Biomasse entfernt, wenn die Flüssigkeit durch mechanisches Trocknen (Abpressen o.ä.) von den Feststoffen getrennt wird.

Die Zerkleinerung findet herkömmlicherweise mit rein mechanisch wirkenden Mühlen, Häckslern, Fräsen o.ä. statt. Deren Energieverbrauch ist relativ hoch und belastet damit die für die Wirtschaftlichkeit der Biomassenutzung wesentliche Energiebilanz negativ.

Die Schneidwerkzeuge derartiger Zerkleinerungseinrichtungen bestehen üblicherweise aus Metall. Sie haben einen erheblichen Verschleiß, müssen also nachgearbeitet (geschliffen) oder ersetzt werden, was die Kosten des Geräteeinsatzes erhöht.

Der Verschleiß bedeutet Abrieb an metallener Substanz, die als Stör- und Schadstoff in die zerkleinerte Biomasse gelangt, also kontraproduktiv ist.

Dabei ist der Verschleiß und dementsprechend der schädliche Abrieb besonders groß, wenn - wie häufig - in der Biomasse Störstoffe wie Steine, Metalle (Hufeisen, Blechdosen oder anderer metallener Unrat) eingeschlossen sind.

Hinzu kommt, dass diese dann nicht nur die Schneidwerkzeuge verschleißen, sondern von diesen ihrerseits zerschlagen oder zerrieben werden, so dass sie als Störstoffe eine intensivere Vermischung mit der Biomasse eingehen und nur noch sehr schwer daraus zu entfernen sind.

Hinzu kommt sogar, dass ihre Zerkleinerung mit metallenem Werkzeug zu Funkenbildung und damit womöglich zu Staubexplosionen bei der Zerkleinerung führt.

Ganz besonders schädlich ist es für die Qualität der Biomasse schließlich auch, wenn in ihr enthaltene Kunststoffabfälle wie Plastiktüten, Joghurtbecher, Kunststoffflaschen o.ä. mit ihr mechanisch zerkleinert werden. Sie werden dann nämlich in kleinste Teile zerlegt und sind dann kaum noch von der Biomasse trennbar und aus ihr entfernbar.

Die herkömmlichen Aufbereitungsverfahren haben im Übrigen den Nachteil, dass verklumpte Biomasse nicht so leicht zerkleinert werden kann, sondern u.U. erst aufgelockert werden muss, was einen weiteren Arbeitsgang bedeutet. Dies gilt umso mehr, als solche Klumpen Störstoffe wie Steine oder Metallteile in sich verbergen können, die ohne Auflösung der Verklumpungen nicht einmal wahrgenommen und nicht abgesammelt werden können, mit der bereits beschriebenen Folge einer Beschädigung der Schneidwerkzeuge.

Der Einsatz von Wasserschneidern für die Gewinnung oder Aufbereitung von Biomasse ist bereits bekannt. So gibt es mit einem Wasserschneider betriebene Gartenhäcksler (DE 20 2004 008 685 U1) und Vorrichtungen zur Gewinnung von Fasern aus Biomasse für die Papierindustrie (US 3,680,796; PL 199667 B1) mittels Wasserschneidern. Ein Verfahren und eine Vorrichtung zur Aufbereitung von Biomasse enthaltenden Haushaltsmüll unter Einsatz eines Wasserschneiders ist aus EP 0 521 685 A1 bekannt.

Hieraus leitet sich die Aufgabe ab, ein großtechnisches Verfahren zur Aufbereitung von Biomasse und eine entsprechende Vorrichtung vorzuschlagen, die im Energieverbrauch wirtschaftlicher sind als die bekannten Verfahren und als die dabei verwendeten herkömmlichen Gerätschaften wie etwa Häcksler oder Mahlwerke, die einen geringeren Verschleiß für die verwendeten Gerätschaften mitsichbringen, so dass diese beispielsweise nicht oder seltener nachgearbeitet werden müssen, die die etwa vorhandenen Störstoffe wie Steine, Eisenmetall o.ä. möglichst unzerkleinert lassen und dadurch im Endeffekt die Herstellung eines kostengünstigeren, umweltfreundlicheren und wirtschaftlicheren Produkts ermöglichen.

Diese Aufgabe lösen ein Verfahren nach Anspruch 1 und eine Vorrichtung nach Anspruch 9. Die jeweiligen Unteransprüche kennzeichnen Einzelheiten des erfindungsgemäßen Verfahrens und der erfindungsgemäßen Vorrichtung.

Der Gegenstand der Anmeldung unterscheidet sich von vorstehend genanntem Stand der Technik in mehreren wesentlichen Merkmalen, insbesondere in der Gestaltung der Zuführeinrichtung und der Anordnung und der Gestaltung des Wasserschneiders.

Biomasse im Sinne der vorliegenden Erfindung umfasst pflanzliche Erzeugnisse wie alle Arten von Biomasseabfällen bzw. Biomassereststoffen. Der Begriff umfasst also jede Art von Grünschnitt beispielsweise Halmgut wie Gras, Getreide, Mais, Schilf, Silagen aber auch getrocknetes Halmgut wie Heu oder Stroh, weiter Laub, Zweige, dünne Äste und auch jede Art pflanzlicher Abfälle, beispielsweise aus der land- forst- und wasserwirtschaftlichen Produktion, wie Algen, Wasserhyazinthen und jede Art pflanzlichen Treibgutes, ebenso wie Trester aus der Wein- oder Bierherstellung, schließlich Landschaftspflegematerial wie Straßenbegleitgrün, landwirtschaftliche Reststoffe sowie alle oben genannten Biomassen als Silagen.

Erfindungsgemäß erfolgt die übliche Zerkleinerung der Biomasse mittels eines Wasserschneiders bzw. Wasserstrahlschneiders. Der Einsatz eines mit einer Flüssigkeit arbeitenden Wasserschneiders führt von vornherein zu einer deutlichen Reduktion des Energieverbrauchs, insbesondere dann, wenn der Schneidstrahldruck auf das jeweils der geschnittenen Biomasse entsprechend geringst mögliche Maß eingestellt wird, was die erfindungsgemäße Vorrichtung möglich macht.

Dies gilt umso mehr, als erfindungsgemäß weiter vorgesehen sein kann, die Schneidvorrichtung überhaupt stillzustellen, wenn die zugeführte Biomasse keine Zerkleinerung erfordert, weil sie bereits auf das erwünschte Maß zerkleinert ist, wie dies etwa bei kurzem Rasenschnitt der Fall sein mag. Da die Anlage dann auch ein bloßes Hindurchfördern der bereits hinreichend zerkleinerten Biomasse realisiert und abgestellt werden kann, führt dies zu einer erheblichen Stromersparnis.

Zu einer weiteren Senkung des Energieverbrauchs führt es, wenn der Schneidstrahldruck jederzeit an die gerade verarbeitete Biomasse angepasst und auf den für deren Schneiden gerade noch erforderlichen geringst möglichen Druck einreguliert werden kann.

Das Arbeiten mit dem geringst möglichen Druck hat zugleich den weiteren erheblichen Vorteil, dass die Biomasse nicht mit präzisen Schnittkanten zerkleinert, sondern stark zerfasert wird, wodurch sich ein besonders weitgehender Aufschluss der Zellstruktur ergibt.

Eine weitere Reduktion des Energieverbrauchs ergibt sich dadurch, dass die Zerkleinerung von Störstoffen wie Steinen, Schrott usw., die in der Biomasse enthalten sein mögen, von vornherein ausgeschlossen werden kann. Dies ist beim Wasserschneiden - anders als beim mechanischen Schneiden mit Metallmessern - ohne Weiteres möglich, weil die Stärke des Schneidstrahls auf das für das hydraulische Schneiden der zugeführten Biomasse nötige Maß begrenzt werden kann, das nicht ausreicht, um auch die Störstoffe zu zerkleinern. Die Störstoffe können dann die Schneideinrichtung unzerstört passieren. Da sie nicht zerkleinert werden, belasten sie die Energiebilanz nicht, und wenn sie - wie etwa weggeworfene Batterien - gar Giftstoffe enthalten, so gehen auch sie nicht in die Biomasse ein.

Dies gilt besonders auch für Störstoffe, die sich in Verklumpungen von Biomasse verbergen. Der Wasserschneider löst die Verklumpungen, die Störstoffe passieren die Zerkleinerungsvorrichtung unzerkleinert und können, etwa beim anschließenden Waschen oder Sieben der Masse, leicht daraus entfernt werden.

Die Störstoffe gehen damit auch nicht in Form kleinster Partikel in die Biomasse ein, können also später unschwer entfernt werden. Sie blockieren andererseits auch nicht die Zerkleinerungsvorrichtung, wie dies bei mit zu geringer Kraft betriebenen starren Messern passieren könnte.

Da keine metallenen Messer eingesetzt werden, findet an dieser Stelle kein Verschleiß statt. Der Eintrag von Abrieb der Werkzeuge in die Biomasse entfällt, so dass die Qualität der Biomasse nicht beeinträchtigt wird.

Dies gilt besonders auch und gerade, wenn die Störstoffe giftige Substanzen enthalten, wie dies etwa bei weggeworfenen Batterien der Fall ist. Auch hier ist es von erheblichem Vorteil, wenn sie die Zerkleinerungsanlage wegen des entsprechend schwach arbeitenden Schneidstrahls unbeschadet passieren und später als Ganzes entfernt werden können
Ein Funkenschlag und die damit verbundenen Risiken entfallen mangels Einsatz von Metallwerkzeugen. Auch entfällt der für ein Nacharbeiten der Werkzeuge sonst erforderliche Aufwand, wodurch die Wirtschaftlichkeit des Verfahrens und der Vorrichtung weiter erhöht wird.

Die Verwendung eines Wasserschneiders hat darüber hinaus eine Reihe von Vorteilen, die beim Einsatz metallener Schneidwerkzeuge fehlen:
So ist es durchaus üblich, die Biomasse vor dem Zerkleinern zu waschen. Dieses Waschen führt einerseits zum Abscheiden von Störstoffen wie Sand, Ruß u.ä., die der Biomasse äußerlich anhaften. Es führt aber vor allem bei der bereits zerkleinerten Biomasse zur Erweichung und zum Aufschwemmen des Materials, wie dies beispielsweise bei trockenem Stroh erwünscht sein kann. Es führt weiter zur Auswaschung des Zellinneren und damit zu dessen erwünschtem Überführen in eine flüssige Phase. Wenn mit dem Wasserschneider gearbeitet wird, wird dieses Auswaschen und Aufschwemmen mit dem Schneiden der Biomasse zugleich erledigt, so dass ein Arbeitsgang eingespart wird.

Dieses Auswaschen der Zellinhaltsstoffe bzw. schädlicher Substanzen führt insbesondere auch zu einer Überführung der wasserlöslichen Substanzen wie Cl, K, Mg etc. in die zum Waschen verwendete Flüssigkeit, mit der sie abgeschieden werden können.

Das Waschen bereits vor dem Zerkleinern hat den Vorteil, dass außen an der Biomasse anhaftende Störstoffe schon vor dem Zerkleinerungsvorgang weggschwemmt werden und nicht mehr in das Gemisch aus Biomasse und Flüssigkeit eingehen, das beim Wasserschneiden entsteht. Es hat aber den weiteren Vorteil, dass beispielsweise trockene Biomasse wie Heu und Stroh bereits vor dem Schneiden aufgeschwemmt, aufgeweicht und damit teilweise erschlossen sowie auch ihr Schneiden erleichtert wird.

Das Schneiden mit Wasserschneider und das Waschen der Biomasse nach dem Zerkleinern gehen mit dem weiteren Vorteil einher, dass sie mit weiteren Behandlungsschritten kombiniert werden können.

Ist etwa eine Vergärung des Gemischs aus Feststoffen und Flüssigkeit erwünscht, das beim Schneiden entsteht, so kann die zum Schneiden verwendete Flüssigkeit zuvor als Prozessflüssigkeit beispielsweise mit den entsprechenden, eine Gärung fördernden Substanzen angereichert werden, oder es wird als Schneidflüssigkeit Prozessflüssigkeit aus einem separaten Gärprozess verwendet.

Ein solches "Animpfen" der Biomasse etwa bereits beim Anfeuchten der Biomasse vor dem Schneiden, beim Schneiden mittels des Wasserschneiders oder danach beim Waschen, beim dem sich beispielsweise Gärbakterien unmittelbar an den Schnittflächen anlagern können, fördert den beabsichtigten Abbau der Biomasse unter Umständen entscheidend und beschleunigt damit die Produktion erheblich.

Eine Erwärmung oder eine Kühlung der Biomasse vor und/oder während der Aufbereitung kann erwünscht sein. Die Biomasse kann dann - gegebenenfalls auch bereits vor dem Zerkleinern - entsprechend temperiert, d.h. erwärmt, gekocht oder gekühlt, werden.

Das Erwärmen der Biomasse kann in der Weise geschehen, dass der Produktstrom mittels angewärmten Brauch-/Frisch-/Prozesswassers oder einer ähnlichen Flüssigkeit in einem Behälter vor oder direkt in der Zuführeinrichtung, etwa in einer vorzugsweise als Zuführschnecke ausgebildeten Zuführspindel, oder mittels Dampf vor dem Schneiden erwärmt wird, dass diese Erwärmung unter oder ohne Druck oder auch im Vakuum etwa auch mittels Austauschflächen an oder direkt in der Zuführeinheit, etwa auch mittels Mikrowellen oder Rotlicht erfolgt.

Ist eine Erwärmung oder Kühlung des Gemischs aus Feststoffen und Flüssigkeit erwünscht, das beim Zerkleinern entsteht, so kann die zum Schneiden im Wasserschneider verwendete Flüssigkeit zuvor als Prozessflüssigkeit entsprechend temperiert, d.h. erwärmt, gekocht oder gekühlt, werden.

Hierbei ist weiter von Vorteil, dass die beim Wasserschneiden verbleibende Restenergie des Schneidstrahls dazu beiträgt, das Gemisch aus Biomasse und Flüssigkeit deutlich zu erwärmen, so dass eine weitere Energiezufuhr oft entbehrlich sein wird.

Dabei wird es sich empfehlen, die Flüssigkeit, nachdem sie den Wasserschneider durchlaufen hat, mit der nunmehr zerkleinerten Biomasse aufzufangen. Es kann dann, soweit es nicht in dem Gemisch aus zerkleinerter Biomasse und Flüssigkeit zur weiteren Bearbeitung verbleiben soll, daraus abgeschieden und - gegebenenfalls nach einer weiteren Aufbereitung wie z.B. Reinigung von Feststoffen, Nachtemperieren - im Kreislauf geführt wieder dem Wasserschneider zugeführt werden. Alternativ zur Kreislaufführung kann auch Frischwasser oder frisches Prozesswasser oder können Mischungen daraus verwendet oder der im Kreis geführten Flüssigkeit beigemischt werden.

Das Schneiden mit Wasserschneider ist aber auch ohne Weiteres möglich, wenn das Ausschwemmen des Zellinneren vermieden werden soll. Dann kann die Biomasse beispielsweise in gefrorenem Zustand, dann etwa auch mit gekühlter Flüssigkeit, geschnitten werden.

Der Wasserschneider arbeitet mit einem Druck im Bereich zwischen 60 und 3000 bar, maximal mit 6000 bar. Normales feuchtes Gras etwa lässt sich mit einem Druck von 200 - 300 bar gut schneiden. Ist die Biomasse härter (beispielsweise Zweige) oder ist sie sehr kompaktiert, so ist ein höherer Schneiddruck vonnöten. Die gängige Biomasse ist mit Drücken von 60 - 600 bar gut zu bewältigen. Bei einem Einsatz mit 600 bar liegt der Energieverbrauch beispielsweise bei 15 - 20 kW, also deutlich niedriger als bei herkömmlicher mechanischer Zerkleinerung mit metallenem Werkzeug.

Der Druck des Schneidstrahls kann an Art und Konsistenz der zu schneidenden Biomasse angepasst werden. Hierzu kann eine Detektion von Art bzw. Konsistenz des Biomassestroms vorgesehen sein. Wenn die zugeführte Biomasse bereits hinreichend kleinteilig ist, kann der Schneidstrahl auch (vorübergehend) ganz abgestellt werden.

Um Störstoffe, d.h. Anhaftungen wie Sand und Ruß oder Steine, Metallteile, Batterien und ähnliche Fehlwürfe aus der Biomasse zu entfernen, wird das im Zerkleinerungsschritt erzeugte Gemisch aus zerkleinerter Biomasse und Flüssigkeit einer Waschanlage zugeführt. Dabei hat das Schneiden mit Flüssigkeitsstrahl den weiteren Vorteil, dass die zum Schneiden verwendete Flüssigkeit die zerkleinerte Biomasse zugleich in die Waschanlage spült und dort verteilt und dass sie dort als Waschflüssigkeit und gegebenenfalls zugleich als Prozessflüssigkeit, beispielsweise um die Biomasse anzumaischen und einen Gärprozess in Gang zu setzen, zur Verfügung steht und/oder zunächst trockene Biomasse pumpfähig macht.

Die Waschanlage ist im einfachsten Fall ein Behälter, gegebenenfalls mit einer Rühreinrichtung, in dem die Biomasse insbesondere im schwebenden Zustand gehalten wird und störende Feststoffe, die leichter als die Flüssigkeit sind, beispielsweise Kunststoffbestandteile, aufschwimmen und abgeschöpft bzw. sonst ausgetragen werden können, während Störstoffe, die schwerer als die Flüssigkeit sind, wie Steine, Metallteile usw., absinken und so ebenfalls von der Biomasse abgeschieden werden können.

Das Schneiden mit Flüssigkeit hat dabei den weiteren Vorteil, dass die für den Betrieb des Wasserschneiders erforderliche Flüssigkeit aus dem Gemisch aus zerkleinerter Biomasse und Flüssigkeit (etwa aus der Wascheinrichtung) entnommen werden kann. Sie muss also nicht jeweils neu bereitgestellt und entsorgt werden, sondern kann in einem geschlossenen System im Kreis geführt werden. Dies hat einerseits den Vorteil, dass die Flüssigkeit bereits weitgehend als Prozessflüssigkeit optimiert, d.h. erwärmt, gekühlt oder mit Prozessmitteln wie Gärförderern angereichert, ist, hier also ein zusätzlicher Aufwand vermieden wird, andererseits auch die mit der Entsorgung derartiger Prozessflüssigkeiten verbundenen Umweltprobleme minimiert werden.

Denn wenn die weitere Bearbeitung der Biomasse eine in bestimmter Weise aufbereitete Flüssigkeit erfordert, kann diese Aufbereitung bereits vor der ersten Verwendung zum Schneiden der Biomasse vorgenommen werden. Die Flüssigkeit wird dann also zuvor gereinigt und/oder temperiert, d.h. erwärmt, gekocht oder gekühlt, und/oder mit prozessrelevanten Substanzen wie etwa gärfördernden Substanzen wie Impfmaterial, Impfsubstrat oder etwa Bakterien angereichert. Bevor die Flüssigkeit im Kreislauf erneut dem Wasserschneider zugeführt wird, kann sie dann nachtemperiert und beispielsweise auch gereinigt werden, damit sie keine Probleme beim Betrieb des Wasserschneiders verursacht.

Unter Prozessflüssigkeit soll im Rahmen dieser Erfindung eine Flüssigkeit verstanden werden, welche während der Aufbereitung der Biomasse anfällt oder eingesetzt wird. Die Prozessflüssigkeit wird häufig auch als Prozesswasser bezeichnet. Sie kann Frischwasser, Abwasser, Brauchwasser, Presswasser als Ergebnis mechanischen Auspressens der Biomasse oder fermentierte Flüssigkeit oder ein Gemisch aus zumindest zwei der genannten Flüssigkeiten sein.

Zusätzlich kann Wasser, Prozesswasser, Impfsubstrat - neben der Zuführung durch den Wasserschneider - bereits durch Zugabe in die Zuführeinheit aufgegeben bzw. dort beigemengt werden, um so schon dort den Prozess des Anmaischens oder Gärens oder jedenfalls ein Aufweichen und Aufschwemmen der Biomasse in Gang zu setzen.

Vor der Weiterverarbeitung der zerkleinerten Biomasse bzw. des Gemischs aus Biomasse und Flüssigkeit muss diese bzw. dieses unter Umständen entwässert werden. Dabei kommt eine Teilentwässerung in Betracht, wenn die Biomasse als feuchte Masse vergoren oder weiterverarbeitet werden soll. Erfordert die weitere Verarbeitung hingegen eine weitgehend trockene Biomasse, so wird eine weitgehende Entwässerung erfolgen. In beiden Fällen wird die Entwässerung soweit wie möglich mechanisch vorgenommen werden, weil sie weniger energieaufwändig ist als eine thermische Trocknung. Eine thermische Trocknung kann bei Bedarf nachgeschaltet werden.

Die mechanische Trennung von Biomasse und Flüssigkeit kann je nach der anschließenden weiteren Bearbeitung durch Absetzen, Abtropfen (Sieben), Zentrifugieren, Pressen o.ä. erfolgen. Die abgeschiedene Flüssigkeit wird - gegebenenfalls nach einer weiteren Aufbereitung wie einer Reinigung oder Nachtemperieren - erneut dem Wasserschneider zugeführt.

Für die Funktion des Wasserschneiders ist es wesentlich, dass die Biomasse der Schneidvorrichtung in einem Zuführschritt dosiert zugeführt wird, d.h. dass die Konsistenz (Art und Dichte) der zugeführten Biomasse entsprechend detektierbar und regelbar und gegebenenfalls auch die Stärke und die Wassermenge des Schneidstrahls in Anpassung an die Konsistenz der zugeführten Biomasse, beispielsweise auch durch Austausch der eingesetzten Düsen, modifizierbar ist.

Deshalb ist erfindungsgemäß eine Dosiereinrichtung mit einer Einrichtung zur Bestimmung von Art, Zusammensetzung und Dichte des Biomassestroms in der Zuführeinrichtung und einer Einrichtung zur Regelung des Zuführgeschwindigkeit der Biomasse zum Wasserschneider sowie der Stärke und Wassermenge des Schneidstrahls vorgesehen, die auch eine Abschaltung des Schneidstrahls für den Fall vorsieht, dass die Biomasse bereits hinreichend zerkleinert ist und nur dosiert zur weiteren Bearbeitung durch die Schneideinrichtung hindurchgefördert werden soll.

Zur Förderung der Biomasse in der Zuführeinrichtung kann auch eine Förderspindel bzw. Förderschnecke vorgesehen sein. Damit die Biomasse zusammengehalten und im Querschnitt der lichten Weite der Schneidvorrichtung des Wasserschneiders angepasst wird, ist die Förderspindel mindestens in ihrem abtriebsseitigen Endbereich von Führungsflächen für die zuzuführende Biomasse umschlossen. Diese Führungsflächen haben in einem solchen Fall beispielsweise die Form eines Aufnahmetrichters für die zugeführte Biomasse, der im abtriebsseitigen Endbereich der Förderspindel in einen diese umschließenden Zylinder oder Konus übergeht.

Zur Zuführeinrichtung kann auch ein Vorratsbehälter/Mischbehälter oder ein Aufgabebehälter gehören, in dem größere Mengen von Biomasse von größenordnungsmäßig 100 m³ oder mehr zur Gewährleistung eines kontinuierlichen Betriebes, etwa auch über Nacht oder über ein Wochenende, vorrätig gehalten und von dort aus in die Zuführung gegeben werden können. Denkbar ist auch, hier zur Vergleichmäßigung des Betriebes eine Mehrzahl von Behältern vorzusehen, die gegebenenfalls sogar automatisiert austauschbar sind.

Der Wasserschneider ist vorzugsweise am abtriebsseitigen Ende der Zuführeinrichtung angeordnet. Er verfügt über mindestens einen Schneidstrahl.

Der Schneidstrahl wirkt mit einer Gegenschneide zusammen. Die Gegenschneide ist am abtriebsseitigen Ende der Zuführeinrichtungen angeordnet. Die Gegenschneide kann eine Gegenschneidleiste sein, die beispielsweise am Endbereich der Führungsflächen ausgebildet ist.

Als Gegenschneide kann auch die zylindrische oder konische Führungsfläche, die den Endbereich der Förderspindel umgibt, eingesetzt werden. Als Gegenschneide wird auch hier in erster Linie die abtriebsseitige Vorderkante eingesetzt bzw. ausgestaltet sein.

Wenn als Gegenschneide eine Gegenschneidleiste eingesetzt wird, wird der Schneidstrahl meist derart hart an der Gegenschneide vorbeigeführt, dass die Biomasse abgeschert wird.

Die Wasserschneideinrichtung kann aber auch so ausgelegt sein, dass der Schneidstrahl im Wesentlichen senkrecht auf eine Gegenfläche, etwa die zylindrischen bzw. konischen Führungsflächen, trifft, die den Endbereich der Zuführspindel umgeben. In diesem Falle kommt es mehr zu einem Quetschen oder einem Zerreißen oder Zerfetzen der Biomasse als zu einem sauberen Abscheren. Im vorgesehenen Anwendungsfall kann aber gerade diese Art der Zerkleinerung erwünscht sein, weil sie zu einem verbesserten Aufschluss der Zellstruktur führt und zugleich verhindert, dass sich beim Abschneiden kompaktierte Biomassescheiben bilden, die dann wieder aufgelöst werden müssten.

Als Gegenschneide kann alternativ zu einer Gegenschneidleiste mindestens ein dem mindestens einen Schneidstrahl entgegengerichteter weiterer Schneidstrahl vorgesehen sein.

Der Schneidstrahl kann oszillierend über und/oder unter und/oder seitlich der Biomasse oder um den zopfförmigen Strom von Biomasse umlaufend geführt werden.

Dabei ist der Wasserschneider vorzugsweise am abtriebsseitigen Ende des Förderbaumes der Förderspindel angeordnet und um dessen Längsachse rotierend derart ausgebildet, dass der Schneidstrahl im Wesentlichen radial nach außen gerichtet umläuft.

Auch in diesem Falle bietet sich an, die Gegenschneide als Gegenschneidleiste auszugestalten und sie insbesondere am abtriebsseitigen Endbereich der Führungsflächen (des die Förderspindel umschließenden Zylinders) auszubilden. Alternativ kann aber auch in diesem Fall anstelle einer Gegenschneidleiste mindestens ein den Strom der Biomasse umlaufender, radial nach innen gerichteter weiterer Schneidstrahl als Gegenschneide vorgesehen sein, dessen Umlauf dann freilich mit dem Umlauf des ersten Schneidstrahls vorzugsweise synchronisiert sein muss. Schneidstrahl und Gegenschneidleiste können aber auch umfangsmäßig zueinander versetzt angeordnet sein.

Alternativ kann der Wasserschneider aber auch in diesem Fall in der Zuführeinrichtung, d.h. innerhalb der konischen oder zylindrischen Führungsflächen des Endbereichs der Zuführeinrichtung angeordnet sein. Dann wird er vorzugsweise in der beschriebenen Weise gegen die Führungsflächen wirken, was für die erfindungsgemäßen Anwendungen wegen der damit verbundenen Art der Zerkleinerung der Biomasse durchaus vorzuziehen sein kann.

Schließlich kann die Zuführeinrichtung auch nur aus einem gebräuchlichen Dosierbehälter bestehen, in dem die Biomasse vorgehalten wird und an dem der Wasserschneider unmittelbar anschließend angeordnet ist, so dass die Biomasse direkt am oder kurz nach und/oder vor dem Austrag aus dem Dosierbehälter mit der Flüssigkeit geschnitten wird. Als Dosier- bzw. Vorlagebehälter kann ein gebräuchlicher Mischer-, Dosierer- oder Vorlagebehälter Verwendung finden, der die Biomasse beispielsweise mittels eines Austragsarms (Sichel) oder einer waagerecht oder senkrecht stehenden Misch- und/oder Austragsschnecke austrägt oder der als Fördereinrichtung ein Schubboden/Kratzboden/Abschiebesystem aufweist. Eine Förderspindel wäre dann am Austrag nicht zwingend erforderlich. Gegebenenfalls kann auch mittels Wasserschneider kurz vor dem Austrag in dem Dosier-Vorlagebehälter geschnitten werden, d.h. der Wasserschneider ist so angeordnet, dass die Biomasse im Bereich vom Austrag des Dosierbehälters geschnitten wird.

Für zahlreiche Anwendungen der erfindungsgemäßen Vorrichtung ist von wesentlicher Bedeutung, dass die Biomasse nach ihrer Zerkleinerung gewaschen bzw. gewässert wird. Dies erleichtert vor allem die Abscheidung von Störstoffen aller Art. Deshalb ist erfindungsgemäß eine dem Wasserschneider nachgeschaltete Wascheinrichtung zum Waschen des Gemischs aus zerkleinerter Biomasse und Flüssigkeit vorgesehen. Diese ist im einfachsten Falle ein Behälter, der das Gemisch aus Biomasse und Flüssigkeit, das beim Zerkleinern entsteht, auffängt.

In der Wascheinrichtung wird die zerkleinerte Biomasse, gegebenenfalls mittels einer Rühreinrichtung, in dem Wasser durchmischt und gewaschen. Leichtere Stoffe wie Plastik schwimmen mit der Biomasse auf und können abgeschöpft oder sonst ausgetragen werden. Schwerere Stoffe lagern am Boden ab und können so ebenfalls leicht ausgetragen werden. Das Durchmischen der Flüssigkeit/Biomasse in der Wascheinrichtung erfolgt vorzugsweise mechanisch, kann aber auch hydraulisch durch Einbringen eines Stromes turbulenter Flüssigkeit oder pneumatisch durch Einbringen von Luft oder eines Gases bzw. Dampfes erfolgen.

Wenn das Gemisch aus Biomasse und Flüssigkeit anschließend in einer mehr oder weniger flüssigen Phase weiterverarbeitet werden soll, kann in der Wascheinrichtung zugleich eine Vorrichtung zur Bestimmung des Flüssigkeitsgehaltes des Gemischs sowie eine Flüssigkeitszu- und/oder Flüssigkeitsabführeinrichtung vorgesehen sein, um das Gemisch auf einen definierten, für die vorgesehene weitere Verarbeitung erforderlichen Flüssigkeitsgehalt einzustellen.

In gleicher Weise kann, wenn eine definierte, für die weitere Bearbeitung bestimmte Temperatur des Gemischs eingehalten werden soll, eine Einrichtung zur Bestimmung der Temperatur des Gemischs und eine Heiz- und/oder Kühleinrichtung zum entsprechenden Temperieren oder Nachtemperieren des Gemischs vorgesehen sein.

Das Erwärmen der Biomasse kann in der Weise geschehen, dass der Produktstrom mittels angewärmten Brauch-/Frisch-/Prozesswassers oder einer ähnlichen Flüssigkeit bereits in einem Behälter vor oder direkt in der Zuführeinrichtung, etwa in der Zuführspindel, oder mittels heißen Dampfes vor dem Schneiden erwärmt wird, wobei diese Erwärmung mit oder ohne Druck oder auch im Vakuum etwa auch über Austauschflächen an oder direkt in der Zuführeinheit, etwa auch mittels Mikrowellen oder Rotlicht erfolgen kann. Auch kann die Erwärmung dadurch bewirkt werden, dass die zum Schneiden im Wasserschneider verwendete Flüssigkeit zuvor als Prozessflüssigkeit entsprechend temperiert, d.h. erwärmt oder gekocht, werden. In gleicher Weise kann eine etwa beabsichtigte Kühlung der Biomasse bewirkt werden.

Einen besonderen Vorteil bietet das Temperieren des Gemisches aus Flüssigkeit und Biomasse durch Einblasen entsprechend temperierter Luft. Die einströmenden Luftblasen sorgen hier nicht nur für eine beschleunigte Temperaturanpassung, sondern sorgen zugleich für eine Durchlockerung des Gemisches.

Für den Fall, dass für die weitere Verarbeitung der Biomasse eine im wesentlichen feste (beispielsweise breiige) Konsistenz erwünscht ist, kann eine der Wascheinrichtung nachgeschaltete Einrichtung zum Abscheiden und Auffangen der Flüssigkeit aus dem Gemisch aus zerkleinerter Biomasse und Flüssigkeit vorgesehen sein. In Fällen, in denen das Waschen des Gemischs aus zerkleinerter Biomasse und Flüssigkeit in der Wascheinrichtung als entbehrlich angesehen wird und für die die weitere Verarbeitung die Entwässerung des Gemischs genügt, kann die Einrichtung zum Abscheiden und Auffangen der Flüssigkeit auch unmittelbar der Zerkleinerungseinrichtung nachgeschaltet werden.

Die Einrichtung zum Abscheiden und Auffangen der Flüssigkeit kann eine einfache Sieb- oder Filtereinrichtung sein. Wenn eine hochgradigere mechanische Entwässerung gewollt ist, wie es etwa für die Herstellung von trockenem Brennstoff zur Verbesserung der Energiebilanz sinnvoll ist, wird als Entwässerungseinrichtung hingegen eine Presse, insbesondere eine Schneckenpresse, eine Zentrifuge oder eine ähnliche hochwirksame Einrichtung zum mechanischen Trocknen zum Einsatz kommen.

Damit die in der Wascheinrichtung oder in der Einrichtung zum Abscheiden und Auffangen von Flüssigkeit anfallende Flüssigkeit dem Wasserschneider zur weiteren Verwendung wieder zugeführt werden kann, ist eine Versorgungsleitung zwischen diesen Anlageteilen vorgesehen. In sie ist eine Einrichtung zur Reinigung bzw. Filtration und zum Nachtemperieren der Flüssigkeit integriert.

Die Erfindung wird im Folgenden an Hand der Zeichnung näher beschrieben. Dabei zeigen:
- **Fig. 1**: die erfindungsgemäße Vorrichtung zur Aufbereitung von Biomasse mit einer als Wasserschneider ausgebildeten Zerkleinerungseinrichtung und zylindrischer Zuführeinrichtung für die Biomasse;
- **Fig. 2 a -c**: die Zuführeinrichtung mit Förderspindel/Förderschnecke und Wasserschneider nach Figur 1 im Querschnitt sowie in einer Ansicht auf ihr abtriebsseitiges Ende mit unterschiedlichen Ausführungsformen des Wasserschneiders; und
- **Fig. 3**: ein weiteres Ausführungsbeispiel einer Zuführeinrichtung mit vorgeschaltetem Dosierbehälter mit integriertem Rührwerk.

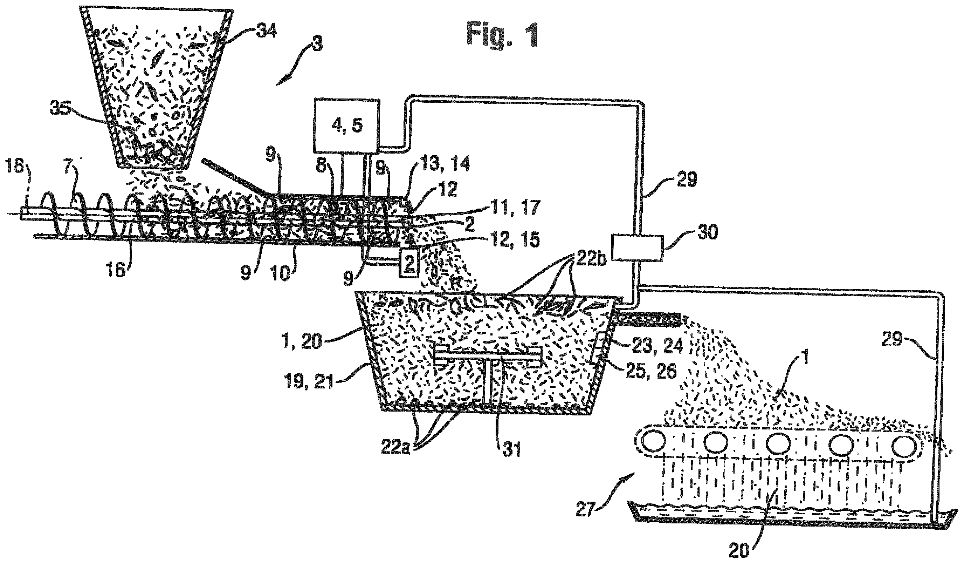

**Fig. 1** zeigt eine Vorrichtung zur Aufbereitung von Biomasse 1 mit einer als Wasserschneider 2 ausgebildeten Zerkleinerungseinrichtung.

Die Vorrichtung verfügt über eine Zuführeinrichtung 3 zur dosierten Zuführung der Biomasse 1 zum Wasserschneider 2 und eine Einrichtung 4 zur Bestimmung von Art, Zusammensetzung und Dichte des Biomassestroms in der Zuführeinrichtung 3 und eine Einrichtung 5 zur Regelung der Zuführgeschwindigkeit der Biomasse 1 zum Wasserschneider 2 sowie zur Regelung der Stärke und Wassermenge des Schneidstrahls 12 nach Maßgabe von Art und Konsistenz der zugeführten Biomasse 1.

Die Zuführeinrichtung 3 wird mit einer Förderspindel 7 betrieben. Die Förderspindel 7 ist mindestens in ihrem abtriebsseitigen Endbereich 8 von Führungsflächen 9 für die zuzuführende Biomasse 1 umschlossen. Diese Führungsflächen 9 bilden sinnvollerweise dort, wo die Aufgabe der Biomasse 1 stattfindet, die Form eines die Zuführung erleichternden Trichters, der sich zum abtriebsseitigen Ende 11 der Zuführeinrichtung 3 hin zu einem die Förderspindel 7 umschließenden Zylinder 10 verengt. Die durch diese Verengung bewirkte Zwangsführung der Biomasse erstreckt sich hier über mehrere Schneckenwindungen, sie soll sich über mindestens eine halbe Schneckenwindung erstrecken.

Die Probleme, die bei der gleichmäßig dosierten Zuführung der Biomasse 1 auftreten, sind dadurch berücksichtigt, dass die Zuführeinrichtung 3 mit einem Aufgabebehälter 34 versehen ist, aus dem die Biomasse 1 mittels der mit Dornfortsätzen oder Graten versehenen Austragswalzen 35 bereits in weitgehend vergleichmäßigter, dosierter Form der Förderspindel 7 zugeführt werden kann.

Der Wasserschneider 2 ist am abtriebsseitigen Ende 11 der Zuführeinrichtung 3 angeordnet. Sein mindestens ein Schneidstrahl 12 wirkt nach Art einer Schere mit einer Gegenschneide 13 zusammen, die als Gegenschneidleiste 14 ausgebildet und am abtriebsseitigen Ende 11 der Zuführeinrichtung 3 angeordnet ist.

Der Wasserschneider 2 sitzt am abtriebsseitigen Ende 11 der Zuführeinrichtung 3 auf dem abtriebsseitigen Ende 17 des Förderbaumes 16 der Förderspindel 7, und sein Strahl 12 wirkt in Rotation um die Längsachse 18 des Förderbaumes 16.

Als Gegenschneide 13 kann wiederum nach Art einer Schere eine Gegenschneidneidleiste 14 vorgesehen sein, die am abtriebsseitigen Ende 11 der Zuführeinrichtung 3, hier des zylindrischen Endbereichs der Führungsflächen 9, 10, gezeigt ist. Alternativ kommt aber als Gegenschneide 13 auch ein weiterer, dem ersten Schneidstrahl 12 entgegengerichteter weiterer Schneidstrahl 12, 15 in Betracht, wie er in der Figur weiter gezeigt ist.

Dem Wasserschneider 2 ist eine Wascheinrichtung 19 zum Waschen des Gemischs aus zerkleinerter Biomasse 2 und Flüssigkeit 20 nachgeschaltet. Diese Wascheinrichtung 19 besteht aus einem Behälter 21, in dem störende Feststoffe 22a, b im Schwimm-/Sinkverfahren abscheidbar sind. Die Zeichnung zeigt, wie sich Störstoffe 22a, die schwerer als die Flüssigkeit sind, d.h. eine höhere Dichte aufweisen, am Behälterboden absetzen, während Störstoffe 22b, die leichter als die Flüssigkeit sind, an der Oberfläche schwimmen. Um die Trennung der verschiedenen Fraktionen von Feststoffen zu fördern, ist in den Behälter 21 ein Rührwerk 31 integriert.

Die Vorrichtung verfügt über eine Messeinrichtung 23, mit der der Flüssigkeitsgehalt des Gemischs aus zerkleinerter Biomasse 1 und Flüssigkeit 20 in der Wascheinrichtung 19 bestimmbar ist, und über eine Flüssigkeitszu- und/oder Flüssigkeitsabführeinrichtung 24, mittels derer der Flüssigkeitsgehalt auf das für die vorgesehene weitere Verarbeitung erforderlich Maß korrigierbar ist.

Weiterhin sind ein Temperaturfühler 25, mittels dessen die Temperatur des Gemisches aus Biomasse 1 und Flüssigkeit 20 bestimmbar ist, sowie eine Heiz- und/oder Kühleinrichtung 26 vorgesehen, mit der das Gemisch für die vorgesehene weitere Bearbeitung nachtemperierbar ist.

Die Vorrichtung weist schließlich eine der Wascheinrichtung 19 nachgeschaltete Einrichtung 27 zum Abscheiden und Auffangen der Flüssigkeit 20 aus dem Gemisch aus zerkleinerter Biomasse 2 und Flüssigkeit 20 auf. Diese ist hier als einfaches Sieb oder Filter ausgebildet. Bei Bedarf kann aber auch vorgesehen sein, sie als - hier nicht näher beschriebene - Presse, Zentrifuge oder eine ähnliche hochwirksame Einrichtung 28 zum mechanischen Trocknen der Biomasse 1 auszubilden.

Zur Versorgung des Wasserschneiders 2 mit der für seinen Betrieb erforderlichen Flüssigkeit 20 ist eine Versorgungsleitung 29 vorgesehen, mittels derer die Flüssigkeit der Wascheinrichtung 19 oder der Einrichtung 27 zum Abscheiden und Auffangen der Flüssigkeit 20 entnommen und dem Wasserschneider 2 zugeleitet bzw. wieder zugeleitet werden kann.

Vorgesehen ist weiter eine Einrichtung 30, mittels derer die in den Wasserschneider 2 zurückzuführende Flüssigkeit 20 reinigbar und nachtemperierbar ist.

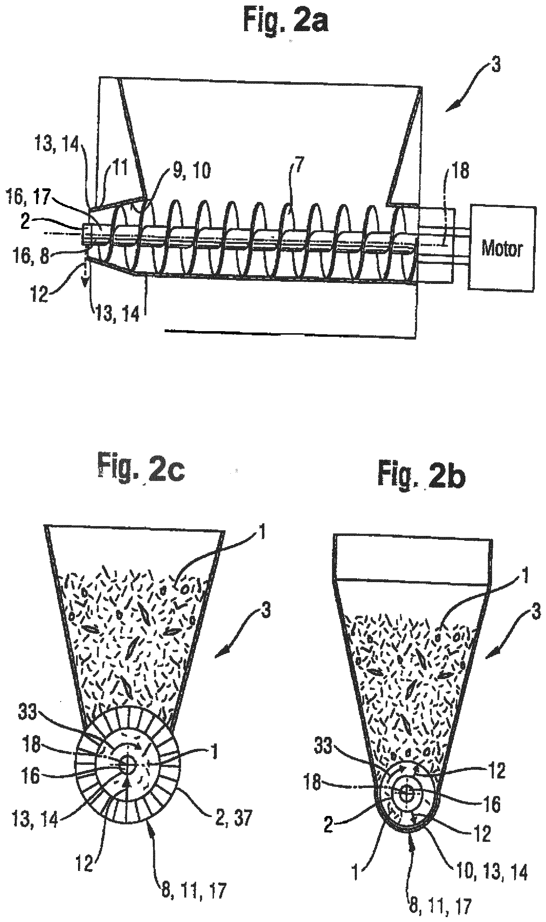

**Fig. 2** **a - c** zeigen die Zuführeinrichtung 3 mit Förderspindel 7 und Wasserschneider 2 nach Fig. 1 im Detail.

**Fig. 2** **a** zeigt die Zuführeinrichtung 3 mit Wasserschneider 2 nach Fig. 1 im Längsschnitt.

Die Zuführeinrichtung 3 weist eine Förderspindel 7 auf, die von Führungsflächen 9 umgeben ist. Die Führungsflächen 9 bilden im abtriebsseitigen Endbereich 11 der Zuführeinrichtung 3 einen Konus 10, der die Förderspindel 7 allseits umschließt. Im rückwärtigen Bereich öffnen sich die Führungsflächen 9 zu einem Aufgabetrichter für die aufzugebende Biomasse 1.

Ein Wasserschneider 2 sitzt am abtriebsseitigen Ende 17 des Förderbaumes 16 der Förderspindel 7. Zum Schneiden rotiert er um die Längsachse 18 des Förderbaumes 16, so dass der Schneidstrahl 12 im Wesentlichen radial nach außen wirkend umläuft.

Der Schneidstrahl 12 wirkt mit einer Gegenschneide 13 zusammen, die als Gegenschneidleiste 14 an dem abtriebsseitigen Ende der zylinderförmigen Führungsfläche 9 der Zuführeinrichtung 3 ausgebildet ist.

**Fig. 2b** zeigt die Zuführeinrichtung 3 mit Wasserschneider 2 nach Fig. 1 in einer Ansicht auf den abtriebsseitigen Endbereich 8, 11, 17 von Zuführeinrichtung 3, Förderspindel 7 und Förderbaum 16.

Der Wasserschneider 2 sitzt am abtriebsseitigen Ende 17 des Förderbaumes 16 der Förderspindel 7. Zum Schneiden der Biomasse 1 rotiert er um die Längsachse 18 des Förderbaumes 16, so dass der Schneidstrahl 12 im Wesentlichen radial nach außen wirkend umläuft.

Der Schneidstrahl 12 wirkt mit einer Gegenschneide 13 zusammen, die als Gegenschneidleiste 14 an dem abtriebsseitigen Ende 11 der zylinderförmigen Führungsflächen 9, 10 der Zuführeinrichtung 3 ausgebildet ist.

Der Pfeil 33 deutet die Rotation der Schneidstrahls 12 an.

**Fig. 2c** zeigt die Zuführeinrichtung 3 mit Wasserschneider 2 nach Fig. 1 in einer Ansicht auf den abtriebsseitigen Endbereich 8, 11, 17 von Zuführeinrichtung 3, Förderspindel 7 und Förderbaum 16.

Der Wasserschneider 2 ist als Düsenring 37 in konzentrischer Anordnung um die Längsachse 18 des Förderbaums 16 am abtriebsseitigen Ende 11 der Zuführeinrichtung 3 angeordnet.

Sein Schneidstrahl 12 wirkt radial nach innen und wirkt dort mit einer Gegenschneide 13 zusammen, die als Gegenschneidleiste 14 am abtriebsseitigen Ende 17 des Förderbaumes 16 ausgebildet ist oder er schneidet direkt auf der Förderspindel 7.

Der Düsenring 37 kann eine Mehrzahl von Schneidstrahlen 12 erzeugen. Dann braucht er zum Schneiden nicht bewegt zu werden, weil bereits die durch die Förderspindel 7 zugeführte Biomasse 1 eine gewisse Drehbewegung ausführt. Es ist aber auch denkbar, mit einem oder wenigen Schneidstrahlen 12 zu arbeiten, die um den Strom der zugeführten Biomasse 1 umlaufend geführt werden.

Der Pfeil 33 deutet die Rotation des Schneidstrahls 12 bzw. auch des Düsenrings 37 oder der rotierend zugeführten Biomasse 1 an.

Wie bereits zu Fig. 1 im Einzelnen erläutert, kann jede der beschriebenen Gegenschneiden 13 statt als Gegenschneidleiste 14 auch als entgegengerichteter weiterer Schneidstrahl 15 ausgebildet sein.

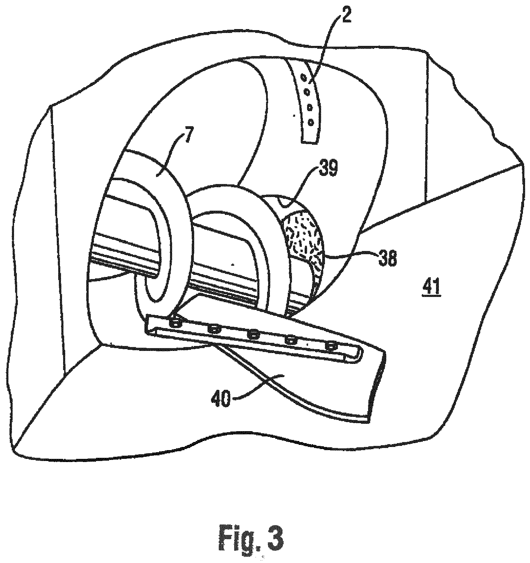

**Fig. 3** zeigt ein weiteres Ausführungsbeispiel einer Zuführeinrichtung mit Wasserschneideinrichtung gemäß der Erfindung. In einem Dosierbehälter 41 ist ein Doppelflügelmesser 40 dargestellt, von dem der Einfachheit halber nur ein Teil eines Flügels gezeigt ist. Dieses Doppelflügelmesser 40 ist am Boden des Dosierbehälters angeordnet und rotiert dabei um eine senkrecht zum Boden des Dosierbehälters 41 angeordnete (nicht dargestellte) Achse. Im Bereich des Überganges vom Boden zur Wandung des Dosierbehälters 41 ist ein im Wesentlichen zylindrischer Ansatz ausgebildet, welcher mit einer Förderspindel 7 durchsetzt ist. Die Förderspindel 7 ist dabei im Wesentlichen senkrecht zur Längserstreckung des im Wesentlichen zylindrisch ausgebildeten Ansatzes angeordnet. Die Förderspindel 7 weist eine umlaufende Förderwendel auf, mittels welcher die Biomasse aus dem Behälter 41 durch eine Austragsöffnung 38 ausgetragen wird, innerhalb welcher die Förderspindel dreht und die Biomasse fördert und welche durch eine Innenkante eines Spindelaustragsstutzens innerhalb der umlaufenden Zylinderfläche des Ansatzes begrenzt ist. Das Doppelflügelmesser 40 am Boden des Dosierbehälters 41 ist dabei so bemessen, dass es bei seiner Rotation eine Kreisfläche überstreicht, deren Peripherie in den durch eine Steigung gebildeten Zwischenraum der Förderschneckenwendel eintaucht, den äußeren Rand der Wendel aber nicht berührt. Durch die Rotation des Doppelflügelmessers 40 wird die Biomasse im Dosierbehälter 41 umgewälzt, gegebenenfalls auch noch weiter zerkleinert und mit einem zusätzlichen am Messer angebrachten schienenartigen Arm in den Zwischenraum der Förderschneckenwendel gefördert, welcher durch die Steigung der an der Förderspindel umlaufenden Wendel gebildet wird. In Folge der Rotation der Förderspindel 7 wird die in den Zwischenraum gelangte Biomasse entlang der Förderspindel, vorbei an der Innenkante des Spindelaustragsstutzens und durch diesen Stutzen hindurch über die Austragsöffnung 38 schließlich aus dem Dosierbehälter 41 herausgefördert.

Gemäß diesem Ausführungsbeispiel ist ein Wasserschneider 2, welcher mehrere Einzeldüsen aufweist, entlang der zylindrischen Innenoberfläche des durch die Förderspindel 7 durchdrungenen Ansatzes angeordnet. Die Düsen des Wasserschneiders sind dabei so gerichtet, dass die Biomasse etwa in dem Bereich geschnitten wird, welcher durch die Peripherie der durch das Doppelflügelmesser 40 überstrichenen Kreisfläche gebildet wird.

Es ist jedoch auch möglich, den Wasserschneider innerhalb der Zylinderfläche des durch die Förderspindel durchdrungenen Ansatzes in dessen Längsrichtung ausgerichtet anzuordnen, so dass die Biomasse in einem solchen Fall quer zur Förderwirkung der Förderspindel 7 geschnitten wird. Es ist außerdem möglich, den Wasserschneider im Wesentlichen entlang der Innenkante 39 des Spindelaustragsstutzens anzuordnen, so dass in einem solchen Fall die Biomasse unmittelbar vor Austritt aus dem Dosierbehälter 41 durch die Austrittsöffnung 38 geschnitten wird. Ebenso ist es denkbar, den Wasserschneider an der nicht bezeichneten umlaufenden Austrittskante der Austragsöffnung 38 anzuordnen, so dass die Biomasse mit dem Wasserschneider unmittelbar am Austritt des Dosierbehälters 41, d.h. also auch nach der Förderschnecke 7 oder noch auf deren Auslaufende geschnitten wird.

Die Zuführeinrichtung gemäß Fig. 3 ist lediglich beispielhaft. Weitere Ausgestaltungen sind durchaus denkbar. So kann beispielsweise das Doppelflügelmesser durch eine oder-je nach Dimensionierung des Dosierbehälters - zwei oder mehrere Schraubspindeln zum Durchmischen der Biomasse im Dosierbehälter ersetzt sein, wobei diese Schraubspindeln mit in Richtung des Bodens vergrößerter Wendelhöhe ausgebildet sein können. Die Schraubspindeln können hinsichtlich ihrer Rotationsachse auch geneigt oder parallel zum Boden des Dosierbehälters angeordnet sein.

Es hat sich gezeigt, dass die mit dem erfindungsgemäßen Verfahren bzw. der erfindungsgemäßen Vorrichtung geschnittene Biomasse, d.h. das Schneiden der Biomasse mittels Wasserschneider, überraschende Synergien mit den an sich bekannten Dosierbehältern bringt, weil dadurch mit dem erfindungsgemäßen Verfahren bzw. der erfindungsgemäßen Vorrichtung die Biomasse noch effektiver aufbereitet werden kann.

### Bezugszeichenliste:

- 1: Biomasse
- 2: Zerkleinerungseinrichtung, Wasserschneider
- 3: Zuführeinrichtung
- 4: Einrichtung zur Bestimmung der Konsistenz der Biomasse
- 5: Einrichtung zur Regelung der Zuführgeschwindigkeit der Biomasse und der Schneidstärke des Wasserschneiders
- 7: Förderspindel/Förderschnecke
- 8: abtriebsseitiger Endbereich der Förderspindel
- 9: Führungsfläche
- 10: Zylinder/Konus
- 11: abtriebsseitiges Ende der Zuführeinrichtung
- 12: Schneidstrahl
- 13: Gegenschneide
- 14: Gegenschneidleiste
- 15: entgegengerichteter weiterer Schneidstrahl
- 16: Förderbaum der Förderspindel 7
- 17: abtriebsseitiges Ende des Förderbaumes 16
- 18: Längsachse des Förderbaumes 16
- 19: Wascheinrichtung
- 20: Flüssigkeit
- 21: Behälter als Wascheinrichtung
- 22 a, b: Feststoffe im Sinne von Störstoffen
- 23: Messeinrichtung zum Bestimmen des Flüssigkeitsgehalts
- 24: Flüssigkeitszu- und/oder Flüssigkeitsabführeinrichtung
- 25: Temperaturfühler
- 26: Heiz- und/oder Kühleinrichtung für Biomasse/Flüssigkeits-Gemisch
- 27: Einrichtung zum Abscheiden und/oder Auffangen der Flüssigkeit
- 28: hochwirksame Einrichtung zum mechanischen Trocknen der Biomasse
- 29: Versorgungsleitung für Wasserschneider
- 30: Einrichtung zum Reinigen und Nachtemperieren der Schneidflüssigkeit
- 31: Rührwerk
- 32: oszillierende Bewegung des Schneidstrahls
- 33: umlaufende Bewegung des Schneidstrahls
- 34: Aufgabebehälter der Zuführeinrichtung
- 35: Austragswalzen/Austragsarme des Aufgabebehälters
- 36: Düsenschlitten
- 37: Düsenring
- 38: Austragsöffnung
- 39: Innenkante des Spindelaustragsstutzens
- 40: Doppelflügelmesser
- 41: Dosierbehälter

## Patentansprüche

1. Verfahren zur großtechnischen Aufbereitung von Biomasse, bei dem die Biomasse mindestens einem Zerkleinerungsschritt unterworfen wird, wobei der mindestens eine Zerkleinerungsschritt mittels eines mit einer Flüssigkeit betriebenen Wasserschneiders erfolgt,
wobei Biomasse dem Wasserschneider in einem Zuführschritt mittels einer Zuführeinrichtung (3) dosiert zugeführt wird,
die eine Förderspindel oder Förderschnecke (7) aufweist,
die zum Zusammenhalten der Biomasse und zur Anpassung ihres Querschnitts an die lichte Weite der Schneidvorrichtung des Wasserschneiders mindestens in ihrem abtriebsseitigen Endbereich (8) von einer Führungsfläche (9) in Form eines Zylinders oder Konus (10) allseits umschlossen ist,
wobei der Wasserschneider (2) am abtriebsseitigen Ende (11) der Zuführeinrichtung (3) angeordnet ist und mit mindestens einer am abtriebsseitigen Ende (11) der Zuführeinrichtung (3) ausgebildeten Gegenschneide (13) zusammenwirkt, wobei ein im Zerkleinerungsschritt erzeugtes Gemisch aus zerkleinerter Biomasse und Flüssigkeit einer Wascheinrichtung (19) zugeführt wird und die Flüssigkeit aus dem Gemisch aus zerkleinerter Biomasse und Flüssigkeit mechanisch abgeschieden wird, oder die Biomasse nach dem Waschen zerkleinert wird und die zerkleinerte Biomasse bzw. das Gemisch aus zerkleinerter Biomasse und Flüssigkeit mechanisch, insbesondere in einer Presse, weiterbearbeitet, d.h. entwässert wird.

2. Verfahren nach Anspruch 1, **dadurch gekennzeichnet, dass** die Flüssigkeit Frischwasser, Brauchwasser einschließlich Abwasser, Prozessflüssigkeit insbesondere aus einer Biogasanlage oder Gülle oder ein Gemisch aus zumindest zwei der genannten Flüssigkeiten ist und/oder im Kreislauf geführt wird.

3. Verfahren nach Anspruch 1 oder 2, **dadurch gekennzeichnet, dass** die Flüssigkeit vor und/oder während des Einsatzes temperiert, insbesondere erwärmt, gekocht oder gekühlt, wird.

4. Verfahren nach einem der vorherigen Ansprüche, **dadurch gekennzeichnet, dass** der Wasserschneider im Bereich zwischen 60 und 600 bar arbeitet.

5. Verfahren nach einem der vorherigen Ansprüche, **dadurch gekennzeichnet, dass** die Biomasse vor und/oder während der Aufbereitung temperiert, insbesondere erwärmt, gekocht, gekühlt oder gefroren, wird.

6. Verfahren nach einem der Ansprüche 1 bis 5 **dadurch gekennzeichnet, dass** die für den Betrieb des Wasserschneiders erforderliche Flüssigkeit aus dem Gemisch aus zerkleinerter Biomasse und Flüssigkeit entnommen wird.

7. Verfahren nach einem der vorherigen Ansprüche, **dadurch gekennzeichnet, dass** die für den Betrieb des Wasserschneiders erforderliche Flüssigkeit vor ihrer Verwendung aufbereitet, insbesondere gereinigt, und/oder temperiert bzw. nachtemperiert, insbesondere erwärmt, gekocht oder gekühlt wird.

8. Verfahren nach Anspruch 7, **dadurch gekennzeichnet, dass** die Flüssigkeit Waschwasser, Abwasser, Brauchwasser oder Presswasser ist.

9. Vorrichtung zur Aufbereitung von Biomasse (1), bestehend aus einer Zerkleinerungseinrichtung (2), die ein mindestens einen Schneidstrahl (12) aufweisender Wasserschneider (2) ist, mit einer Zuführeinrichtung (3) zur dosierten Zuführung der Biomasse (1) zum Wasserschneider (2), die eine Förderspindel oder Förderschnecke (7) aufweist,
die zum Zusammenhalten der Biomasse und zur Anpassung ihres Querschnitts an die lichte Weite der Schneidvorrichtung des Wasserschneiders (2)mindestens in ihrem abtriebsseitigen Endbereich (8) von einer Führungsfläche (9) in Form eines Zylinders oder Konus (10) allseits umschlossen ist,
wobei der Wasserschneider (2) am abtriebsseitigen Ende (11) der Zuführeinrichtung (3) angeordnet ist und mit mindestens einer am abtriebsseitigen Ende (11) der Zuführeinrichtung (3) ausgebildeten Gegenschneide (13) zusammenwirkt.

10. Vorrichtung nach Anspruch 9, **dadurch gekennzeichnet, dass** zur dosierten Zuführung der Biomasse eine Dosiereinrichtung mit einer Einrichtung (4) zur Bestimmung von Art, Zusammensetzung und Dichte der zugeführten Biomasse (1) in der Zuführeinrichtung (3) und einer Einrichtung (5) zur Regelung der Zuführgeschwindigkeit der Biomasse (1) zum Wasserschneider (2) sowie der Stärke des Schneidstrahls (12) vorgesehen ist.

11. Vorrichtung nach Anspruch 9 oder 10, **dadurch gekennzeichnet, dass** die Gegenschneide (13) eine Gegenschneidleiste (14) ist.

12. Vorrichtung nach Anspruch 9 oder 10, **dadurch gekennzeichnet, dass** die Gegenschneide (13) ein dem Schneidstrahl (12) entgegengerichteter weiterer Schneidstrahl (15) ist.

13. Vorrichtung nach einem der Ansprüche 9 bis 12, **dadurch gekennzeichnet, dass** der Schneidstrahl (12) oszillierend über und/oder unter und/oder seitlich der Biomasse (1) und/oder um einen zugeführten Strom der Biomasse (1) umlaufend geführt wird.

14. Vorrichtung nach Anspruch 12 oder 13, **dadurch gekennzeichnet, dass** mehrere Schneidstrahlen (12, 15) als Schneiden (12) und Gegenschneiden (15) zueinander entgegengesetzt geführt werden.

15. Vorrichtung nach einem der Ansprüche 9 bis 14, **dadurch gekennzeichnet, dass** der Wasserschneider (2) am abtriebsseitigen Ende (17) eines Förderbaumes (16) der Förderspindel (7) um dessen Längsachse (18) rotierend derart ausgebildet ist, dass der Schneidstrahl (12, 15) im Wesentlichen radial nach außen gerichtet ist.

16. Vorrichtung nach Anspruch 9 oder 10, **dadurch gekennzeichnet, dass** die Zuführeinrichtung (3) einen Dosierbehälter aufweist, im Bereich von dessen Austrag die Biomasse geschnitten wird.

17. Vorrichtung nach Anspruch 15 oder 16, **dadurch gekennzeichnet, dass** als Gegenschneide (13) ein radial nach innen gerichteter, den Strom der Biomasse (1) synchron zum Schneidstrahl (12) umlaufender, entgegengerichteter Schneidstrahl (15, 12) vorgesehen ist.

18. Vorrichtung nach einem der Ansprüche 9 bis 17, **gekennzeichnet durch** eine dem Wasserschneider (2) nachgeschaltete Wascheinrichtung (19) zum Waschen eines Gemischs aus zerkleinerter Biomasse (2) und Flüssigkeit (20).

19. Vorrichtung nach Anspruch 18, **dadurch gekennzeichnet, dass** die Wascheinrichtung (19) ein Behälter (21) ist, in dem nicht zur Energiegewinnung vorgesehene Feststoffe (22a, b) im Schwimm-/Sinkverfahren abscheidbar sind.

20. Vorrichtung nach Anspruch 18 oder 19, bei der der Flüssigkeitsgehalt des Gemischs aus zerkleinerter Biomasse (1) und Flüssigkeit (20) in der Wascheinrichtung (19) mittels einer Messeinrichtung (23) bestimmbar und mittels einer Flüssigkeitszu- und/oder Flüssigkeitsabführeinrichtung (24) auf ein definiertes Maß korrigierbar ist.

21. Vorrichtung nach einem der Ansprüche 17 bis 20, bei der die Temperatur des Gemischs aus Biomasse (1) und Flüssigkeit (20) mittels Temperaturfühler (25) bestimmbar und mittels einer Heiz- und/oder Kühleinrichtung (26) auf ein definiertes Maß korrigierbar ist.

22. Vorrichtung nach einem der Ansprüche 18 bis 21, **gekennzeichnet durch** eine dem Wasserschneider (2) oder der Wascheinrichtung (19) nachgeschaltete Einrichtung (27) zum Abscheiden und Auffangen der Flüssigkeit (20) aus dem Gemisch aus zerkleinerter Biomasse (2) und Flüssigkeit (20).

23. Vorrichtung nach Anspruch 22, **dadurch gekennzeichnet, dass** die Einrichtung (27) zum Abscheiden und Auffangen der Flüssigkeit eine Presse, Zentrifuge oder eine ähnliche hochwirksame Einrichtung (28) zum mechanischen Trocknen der Biomasse (1) aufweist.

24. Vorrichtung nach einem der Ansprüche 18 bis 23, **gekennzeichnet durch** eine Versorgungsleitung (29), mittels derer die für den Betrieb des Wasserschneiders (2) erforderliche Flüssigkeit (20) der Wascheinrichtung (19) oder der Einrichtung (27) zum Abscheiden und Auffangen der Flüssigkeit entnehmbar und dem Wasserschneider (2) zuleitbar ist.

25. Vorrichtung nach Anspruch 24, **gekennzeichnet durch** eine Einrichtung, mittels derer die in den Wasserschneider (2) zurückzuführende Flüssigkeit (20) reinigbar und temperierbar oder nachtemperierbar ist.

## Claims

1. Method for large-scale processing of biomass, in which the biomass is subjected to at least one comminution step,
wherein the at least one comminution step is carried out by means of a water cutter operated with a liquid,
wherein biomass is metered into the water cutter in a feed step by means of a feed apparatus (3),
which has a conveyor spindle or conveyor screw (7), which, in order to hold together the biomass and to adapt its cross section to the clear width of the cutting device of the water cutter, is enclosed on all sides, at least in its output-side end region (8), by a guide surface (9) in the form of a cylinder or cone (10),
wherein the water cutter (2) is arranged at the output end (11) of the feed apparatus (3) and cooperates with at least one mating cutter (13) formed at the output end (11) of the feed apparatus (3), wherein a mixture of comminuted biomass and liquid formed in the comminution step is fed to a washing apparatus (19) and the liquid is separated out mechanically from the mixture of comminuted biomass and liquid, or the biomass is comminuted after the washing and the comminuted biomass or the mixture of comminuted biomass and liquid is processed further, i.e. dewatered mechanically, in particular in a press.

2. Method according to Claim 1, **characterized in that** the liquid is fresh water, service water including waste water, process liquid, in particular from a biogas plant, or manure or a mixture of at least two of the aforementioned liquids and/or is circulated.

3. Method according to Claim 1 or 2, **characterized in that** the liquid is temperature-controlled before and/or during use, in particular heated, boiled or cooled.

4. Method according to one of the preceding claims, **characterized in that** the water cutter operates in the range between 60 and 600 bar.

5. Method according to one of the preceding claims, **characterized in that** the biomass is temperature-controlled before and/or during the processing, in particular heated, boiled, cooled or frozen.

6. Method according to one of Claims 1 to 5, **characterized in that** the liquid required for the operation of the water cutter is taken from the mixture of comminuted biomass and liquid.

7. Method according to one of the preceding claims, **characterized in that** the liquid required for the operation of the water cutter is processed before its use, in particular cleaned, and/or temperature-controlled or has its temperature re-adjusted, in particular heated, boiled or cooled.

8. Method according to Claim 7, **characterized in that** the liquid is washing water, waste water, service water or press water.

9. Device for processing biomass (1), comprising a comminution apparatus (2), which is a water cutter (2) having at least one cutting jet (12), having a feed apparatus (3) from metering the biomass (1) to the water cutter (2), which has a conveyor spindle or conveyor screw (7) which, to hold together the biomass and to adapt its cross section to the clear width of the cutting device of the water cutter (2), is enclosed on all sides, at least in its output-side end region (8), by a guide surface (9) in the form of a cylinder or cone (10),
wherein the water cutter (2) is arranged at the output end (11) of the feed apparatus (3) and cooperates with at least one mating cutter (13) formed at the output end (11) of the feed apparatus (3).

10. Device according to Claim 9, **characterized in that** for the metered feeding of the biomass, a metering apparatus comprising an apparatus (4) for determining the type, composition and density of the biomass (1) fed in is provided in the feed apparatus (3), and an apparatus (5) for regulating the feed rate of the biomass (1) to the water cutter (2) and the intensity of the cutting jet (12) is provided.

11. Device according to Claim 9 or 10, **characterized in that** the mating cutter (13) is a mating cutter bar (14).

12. Device according to Claim 9 or 10, **characterized in that** the mating cutter (13) is a further cutting jet (15) directed counter to the cutting jet (12).

13. Device according to one of Claims 9 to 12, **characterized in that** the cutting jet (12) is guided in an oscillating manner over and/or under and/or at the side of the biomass (1) and/or circumferentially about a stream of the biomass (1) that is fed in.

14. Device according to Claim 12 or 13, **characterized in that** multiple cutting jets (12, 15) are guided counter to one another as cutters (12) and mating cutters (15).

15. Device according to one of Claims 9 to 14, **characterized in that** the water cutter (2) is formed at the output end (17) of a conveyor tree (16) of the conveyor spindle (7) so as to rotate about its longitudinal axis (18) in such a way that the cutting jet (12, 15) is directed substantially radially outwards.

16. Device according to Claim 9 or 10, **characterized in that** the feed apparatus (3) has a metering container, the biomass being cut in the region of its discharge.

17. Device according to Claim 15 or 16, **characterized in that** the mating cutter (13) provided is an oppositely directed cutting jet (15, 12) directed radially inwards and circulating around the stream of the biomass (1) synchronously with the cutting jet (12).

18. Device according to one of Claims 9 to 17, **characterized by** a washing apparatus (19) arranged downstream of the water cutter (2) to wash a mixture of comminuted biomass (2) and liquid (20).

19. Device according to Claim 18, **characterized in that** the washing apparatus (19) is a container (21), in which solids (22a, b) not provided for energy recovery can be separated out in the flotation/precipitation process.

20. Device according to Claim 18 or 19, in which the liquid content of the mixture of comminuted biomass (1) and liquid (20) in the washing apparatus (19) can be determined by means of a measuring apparatus (23), and can be corrected to a defined extent by means of a liquid feed and/or liquid discharge apparatus (24).

21. Device according to one of Claims 17 to 20, in which the temperature of the mixture of biomass (1) and liquid (20) can be determined by means of temperature sensors (25) and corrected to a defined extent by means of a heating and/or cooling apparatus (26).

22. Device according to one of Claims 18 to 21, **characterized by** an apparatus (27), connected downstream of the water cutter (2) or the washing apparatus (19), for separating and collecting the liquid (20) from the mixture of comminuted biomass (2) and liquid (20).

23. Device according to Claim 22, **characterized in that** the apparatus (27) for separating and collecting the liquid has a press, centrifuge or a similar highly effective apparatus (28) for mechanically drying the biomass (1).

24. Device according to one of Claims 18 to 23, **characterized by** a supply line (29), by means of which the liquid (20) required for the operation of the water cutter (2) can be taken from the washing apparatus (19) or the apparatus (27) for separating and collecting the liquid and fed to the water cutter (2).

25. Device according to Claim 24, **characterized by** an apparatus by means of which the liquid (20) to be fed back into the water cutter (2) can be cleaned and temperature-controlled or have its temperature readjusted.

## Revendications

1. Procédé de préparation à l'échelon industriel d'une biomasse, dans lequel on soumet la biomasse au moins à une étape de fragmentation,
dans lequel ladite au moins une étape de fragmentation est effectuée au moyen d'une machine de découpage au jet d'eau fonctionnant avec un liquide,
dans lequel on fournit la biomasse de façon dosée à la machine de découpage au jet d'eau dans une étape de fourniture au moyen d'un dispositif de fourniture (3),
qui présente une broche de transport ou une vis sans fin de transport (7),
qui est entouré de tous les côtés par une surface de guidage (9) en forme de cylindre ou de cône (10) au moins dans sa région d'extrémité (8) côté sortie pour contenir la biomasse et adapter sa section transversale à la largeur libre du dispositif de coupe de la machine de découpage au jet d'eau,
dans lequel la machine de découpage au jet d'eau (2) est disposée à l'extrémité (11) côté sortie du dispositif de fourniture (3) et coopère avec au moins un couteau opposé (13) formé à l'extrémité (11) côté sortie du dispositif de fourniture (3), dans lequel un mélange de biomasse fragmentée et d'eau produit à l'étape de fragmentation est fourni à un dispositif de lavage (19) et le liquide provenant du mélange de biomasse fragmentée et de liquide est séparé par voie mécanique, ou la biomasse est fragmentée après le lavage et la biomasse fragmentée ou le mélange de biomasse fragmentée et de liquide est retraité, c'est-à-dire déshydraté, par voie mécanique, en particulier dans une presse.

2. Procédé selon la revendication 1, **caractérisé en ce que** le liquide est l'eau fraîche, l'eau utilitaire y compris l'eau usée, un liquide de traitement, en particulier provenant d'une installation de biogaz, ou du purin ou un mélange d'au moins deux des liquides précités et/ou est fourni en circuit.

3. Procédé selon la revendication 1 ou 2, **caractérisé en ce que** le liquide est mis à température avant et/ou pendant l'emploi, en particulier chauffé, bouilli ou refroidi.

4. Procédé selon l'une quelconque des revendications précédentes, **caractérisé en ce que** la machine de découpage au jet d'eau fonctionne dans une plage comprise entre 60 et 600 bars.

5. Procédé selon l'une quelconque des revendications précédentes, **caractérisé en ce que** la biomasse est mise à température avant et/ou pendant la préparation, en particulier chauffée, bouillie, refroidie ou gelée.

6. Procédé selon l'une quelconque des revendications 1 à 5, **caractérisé en ce que** le liquide nécessaire pour le fonctionnement de la machine de découpage au jet d'eau est prélevé dans le mélange de biomasse fragmentée et de liquide.

7. Procédé selon l'une quelconque des revendications précédentes, **caractérisé en ce que** le liquide nécessaire pour le fonctionnement de la machine de découpage au jet d'eau est préparé avant son utilisation, en particulier nettoyé et/ou en mis à température ou remis à température, en particulier chauffé, bouilli ou refroidi.

8. Procédé selon la revendication 7, **caractérisé en ce que** le liquide est de l'eau de lavage, de l'eau usée, de l'eau utilitaire ou de l'eau de presse.

9. Dispositif de préparation de biomasse (1), se composant d'un dispositif de fragmentation (2), qui est une machine de découpage au jet d'eau (2) présentant au moins un jet de coupe (12), avec un dispositif de fourniture (3) pour la fourniture dosée de la biomasse (1) à la machine de découpage au jet d'eau (2), qui présente une broche de transport ou une vis sans fin de transport (7),
qui est entouré de tous les côtés par une surface de guidage (9) en forme de cylindre ou de cône (10) au moins dans sa région d'extrémité (8) côté sortie pour contenir la biomasse et adapter sa section transversale à la largeur libre du dispositif de coupe de la machine de découpage au jet d'eau (2),
dans lequel la machine de découpage au jet d'eau (2) est disposée à l'extrémité (11) côté sortie du dispositif de fourniture (3) et coopère avec au moins un couteau opposé (13) formé à l'extrémité (11) côté sortie du dispositif de fourniture (3).

10. Dispositif selon la revendication 9, **caractérisé en ce qu'**il est prévu, pour la fourniture dosée de la biomasse, un dispositif de dosage avec un dispositif (4) pour la détermination de la nature, de la composition et de la densité de la biomasse fournie (1) dans le dispositif de fourniture (3) et avec un dispositif (5) pour la régulation de la vitesse de fourniture de la biomasse (1) à la machine de découpage au jet d'eau (2) ainsi que de l'intensité du jet de coupe (12).

11. Dispositif selon la revendication 9 ou 10, **caractérisé en ce que** le couteau opposé (13) est une lame (14) de couteau opposé.

12. Dispositif selon la revendication 9 ou 10, **caractérisé en ce que** le couteau opposé (13) est un autre jet de coupe (15) dirigé en opposition au jet de coupe (12).

13. Dispositif selon l'une quelconque des revendications 9 à 12, **caractérisé en ce que** le jet de coupe (12) est guidé de façon oscillante au-dessus et/ou en dessous et/ou sur le côté de la biomasse (1) et/ou tout autour d'un flux fourni de la biomasse (1).

14. Dispositif selon la revendication 12 ou 13, **caractérisé en ce que** plusieurs jets de coupe (12, 15) sont guidés en opposition les uns aux autres comme couteaux (12) et couteaux opposés (15).

15. Dispositif selon l'une quelconque des revendications 9 à 14, **caractérisé en ce que** la machine de découpage au jet d'eau (2) est réalisée à l'extrémité (17) côté sortie d'un arbre de transport (16) de la broche de transport (7) de façon rotative autour de l'axe longitudinal (18) de celui-ci, de telle manière que le jet de coupe (12, 15) soit dirigé sensiblement radialement vers l'extérieur.

16. Dispositif selon la revendication 9 ou 10, **caractérisé en ce que** le dispositif de fourniture (3) présente un récipient de dosage, dans la région de la sortie duquel la biomasse est tranchée.

17. Dispositif selon la revendication 15 ou 16, **caractérisé en ce qu'**il est prévu comme couteau opposé (13) un jet de coupe (15, 12) dirigé radialement vers l'intérieur, orienté en sens contraire au flux de la biomasse (1) et entourant celui-ci de manière synchrone par rapport au jet de coupe (12).

18. Dispositif selon l'une quelconque des revendications 9 à 17, **caractérisé par** un dispositif de lavage (19) installé après la machine de découpage au jet d'eau (2) pour le lavage d'un mélange de biomasse fragmentée (2) et de liquide (20).

19. Dispositif selon la revendication 18, **caractérisé en ce que** le dispositif de lavage (19) est un récipient (21), dans lequel des matières solides (22a, b) non prévues pour la production d'énergie peuvent être séparées par un procédé de flottation/décantation.

20. Dispositif selon la revendication 18 ou 19, dans lequel la teneur en liquide du mélange de biomasse fragmentée (1) et de liquide (20) peut être déterminée dans le dispositif de lavage (19) au moyen d'un dispositif de mesure (23) et peut être corrigée à un niveau défini au moyen d'un dispositif d'addition et/ou d'extraction de liquide (24).

21. Dispositif selon l'une quelconque des revendications 17 à 20, dans lequel la température du mélange de biomasse (1) et de liquide (20) peut être déterminée au moyen de capteurs de température (25) et peut être corrigée à un niveau défini au moyen d'un dispositif de chauffage et/ou de refroidissement (26).

22. Dispositif selon l'une quelconque des revendications 18 à 21, **caractérisé par** un dispositif (27) installé après la machine de découpage au jet d'eau (2) ou le dispositif de lavage (19) pour la séparation et la collecte du liquide (20) provenant du mélange de biomasse fragmentée (2) et de liquide (20).

23. Dispositif selon la revendication 22, **caractérisé en ce que** le dispositif (27) pour la séparation et la collecte du liquide présente une presse, une centrifugeuse ou un dispositif (28) similaire à haut rendement pour le séchage mécanique de la biomasse (1).

24. Dispositif selon l'une quelconque des revendications 18 à 23, **caractérisé par** une conduite d'alimentation (29), au moyen de laquelle le liquide (20) nécessaire pour le fonctionnement de la machine de découpage au jet d'eau (2) peut être prélevé dans le dispositif de lavage (19) ou dans le dispositif (27) pour la séparation et la collecte du liquide et peut être fourni à la machine de découpage au jet d'eau (2).

25. Dispositif selon la revendication 24, **caractérisé par** un dispositif, au moyen duquel le liquide (20) à renvoyer dans la machine de découpage au jet d'eau (2) peut être nettoyé et mis à température ou remis à température.
